# EUROPÄISCHE PATENTSCHRIFT

(11) **EP 4 074 348 B1**
(45) Veröffentlichungstag und Bekanntmachung des Hinweises auf die Patenterteilung: **22.10.2025**
(21) Anmeldenummer: 22020114.9
(22) Anmeldetag: 17.03.2022
(51) Int. Cl.: A61L 9/20

(54) **DESINFEKTIONSVORRICHTUNG**
DISINFECTION DEVICE
DISPOSITIF DE DÉSINFECTION

(30) Priorität: 18.03.2021 DE 102021106740
(43) Veröffentlichungstag der Anmeldung: 19.10.2022
(73) Patentinhaber: Schaeffler, Heinrich, 64720 Michelstadt (DE)
(72) Erfinder: Schaeffler, Heinrich, 64720 Michelstadt (DE)
(74) Vertreter: Baumann, Rüdiger Walter

(56) Entgegenhaltungen:
- EP-A1- 2 567 713
- EP-A1- 2 926 838
- WO-A1-2009/013507
- WO-A1-2014/122185

## Beschreibung

Die Erfindung betrifft das technische Gebiet der UV-Desinfektion von Fluiden.

EP 2 567 713 A1 offenbart den Gegenstand des Oberbegriffs des unabhängigen Anspruchs 1.

Es sind zahlreiche pathogene Keime bekannt, die Luft und Wasser kontaminieren und zu Erkrankungen führen können, wenn die Keime durch den Menschen aufgenommen werden. Es handelt sich hierbei vor allem um Viren und Bakterien. Diese werden in Luft und Wasser mitgeführt und von Menschen über den Mund-Rachenraum oder Verletzungen, wie beispielsweise offene Wunden, aufgenommen. Ausschlaggebend dafür, dass die aufgenommenen Keime zu einer schwerwiegenden Erkrankung führen, ist die in Luft bzw. Wasser mitgeführte Keimmenge. Erreicht diese einen kritischen Wert, der durch die körpereigene Abwehr nicht reduziert werden kann, kommt es zu einer Erkrankung. Insbesondere in der neueren Zeit sind Viren aufgetreten, die über die Atemluft aber auch über Flüssigkeiten aufgenommen werden und schwerwiegende Erkrankungen, insbesondere der Atemwege, hervorrufen. Daneben sind jedoch auch bereits seit langem andere Keime, wie beispielsweise Bakterien bekannt, die über die Luft oder aufgenommene Flüssigkeiten dem Körper zugeführt, dort schwerwiegende Erkrankungen, beispielsweise des Verdauungstraktes hervorrufen.

Um dem entgegenzuwirken, sind aus dem Stand der Technik zahlreiche Desinfektionsmöglichkeiten bekannt. So hat beispielsweise UV-Licht eine abtötende Wirkung auf Pathogene. Gasförmige oder flüssige Substanzen werden mit UV-Licht bestrahlt, um die in den jeweiligen Fluiden mitgeführten Keime abzutöten bzw. deren Zahl auf ein subpathogenes Niveau abzusenken.

Nachteilig bei bekannten Desinfektionsvorrichtungen, die UV-Licht als Desinfektionsmittel verwenden ist, dass bei längerem Gebrauch eine starke Verschmutzung der die UV-Strahlen emittierenden Einheiten stattfindet, da sich in den Fluiden mitgeführte Schmutzpartikel, wie beispielsweise Stäube, auf den UV-Strahlern ablagern bzw. an diesen anhaften. Eine Reinigung der UV-Strahler gestaltet sich oftmals schwierig bis unmöglich. Dies resultiert oftmals in einer durch die Verschmutzung hervorgerufenen Reduzierung der Desinfektionsleistung und macht nicht selten den gesamten Austausch der UV-Einheiten notwendig, um deren Wirkung wiederherzustellen bzw. dauerhaft sicherzustellen.

Aufgabe der Erfindung ist es, eine Desinfektionsvorrichtung zur Verfügung zu stellen, in der eine Verschmutzung der UV-Einheit verhindert, deren Lebensdauer erhöht und ein gleichbleibend hohes Niveau der Desinfektionsleistung zur Verfügung gestellt wird.

Diese Aufgabe wird gelöst durch eine Desinfektionsvorrichtung gemäß Anspruch 1.

Vorteilhafte Weiterbildungen der Erfindung sind Gegenstand der abhängigen Ansprüche.

Die erfindungsgemäße Desinfektionsvorrichtung dient zur Desinfektion von Fluiden. Bei Letzteren handelt es sich um flüssige oder gasförmige Substanzen, wie beispielsweise Wasser oder Luft. Die Vorrichtung ist derart aufgebaut, dass das zu desinfizierende Fluid in einem ersten Bereich in die Vorrichtung eintritt und in einem vom ersten Bereich beabstandeten oder abgewandten zweiten Bereich aus der Vorrichtung austritt. Zwischen erstem und zweitem Bereich ist ein von dem Fluid durchströmbarer Fluidleitungsabschnitt vorgesehen, dem wenigstens eine UV-Strahlung im keimhemmenden bzw. abtötenden Wellenlängenbereich abstrahlende UV-Einheit, bevorzugt eine UV-LED Einheit zur Desinfektion des Fluids zugeordnet ist. Eine UV-LED Einheit hat den Vorteil einer geringeren Wärmeentwicklung und gleichmäßigeren UV-Abstrahlung. Auch ist eine UV-LED Einheit einfacher zu skalieren und zu steuern. Dennoch umfasst die Erfindung sämtliche Möglichkeiten des Standes der Technik zur Ausbildung von UV-Einheiten. In der Beschreibung sowie den Ansprüchen wird die Bezeichnung UV-Einheit verwendet. Dieser Begriff umfasst sämtliche Ausgestaltungsmöglichkeiten von UV-abstrahlenden Einheiten. Um eine ausreichend gute, die Keimlast reduzierende Wirkung der von der UV-Einheit abgestrahlten UV-Strahlung zu erreichen, ist der Fluidleitungsabschnitt transparent und durchlässig für UV-Strahlung ausgebildet. Der Fluidleitungsabschnitt ist dabei derart in der Vorrichtung angeordnet, dass eine vollständige Abschirmung der UV-Einheit gegenüber dem durchströmenden Fluid erreicht wird. Das Fluid kommt somit während des Durchtritts durch die Desinfektionsvorrichtung nie unmittelbar mit der UV-Einheit bzw. den dort vorgesehenen UV-Strahlern in Kontakt. Dennoch erlaubt die Desinfektionsvorrichtung eine Desinfektion des Fluids mittels UV-Strahlung, dies jedoch ohne Berührung der UV-Einheit beim Durchströmen des Fluidleitungsabschnittes. Im Fluidstrom mitgeführte Verschmutzungen, beispielsweise Partikel oder sonstige Verunreinigungen können sich daher nicht auf der UV-Einheit absetzen bzw. an dieser anhaften und dadurch deren Wirkungsgrad reduzieren.

Der Fluidleitungsabschnitt ist dabei aus einem für UV-Strahlung durchlässigen Folienmaterial gebildet und aus der Vorrichtung entnehmbar ausgebildet. Durch Verwendung eines Folienmaterial kann eine Anpassung an verschiedene Geometrie in der Desinfektionsvorrichtung bzw. der UV-Einheit in einfacher Art und Weise erzielt werden. Darüber hinaus ist das Folienmaterial leicht und benötigt wenig Raum in der Desinfektionsvorrichtung. Auch ist die Anbringung des Folienmaterial sehr einfach. Das Folienmaterial weist dabei bevorzugt eine Materialstärke von zwischen 60 µm und 300 µm, insbesondere von 150 µm auf, ohne die Erfindung auf diesen Bereich zu beschränken. Selbstverständlich können auch Folien mit größerer oder geringerer Materialstärke eingesetzt werden.

Als besonders vorteilhaft wird angesehen, wenn das Folienmaterial eine Durchlässigkeit für die von der UV-Einheit abgegebenen UV-Strahlung von zwischen 80% und 99% aufweist. Hierdurch kann eine ausreichend gute Desinfektionsleistung gewährleistet werden, die sicherstellt, dass mit der erfindungsgemäßen Desinfektionsvorrichtung behandelte Fluide frei von Keimen sind bzw. eine auf ein unschädliches Maß reduzierte Keimzahl aufweisen.

Als besonders vorteilhaft erweist es sich, wenn das Folienmaterial selbstreinigend ausgebildet ist. Dies bedeutet, dass das Folienmaterial derart ausgestaltet ist, dass es eine besonders geringe Benetzbarkeit mit Flüssigkeit aufweist und/oder eine verringerte Anhaftung von Schmutzpartikeln zulässt. Diese auch als Lotuseffekt bekannte Eigenschaft der in der erfindungsgemäßen Desinfektionsvorrichtung eingesetzten Folie stellt sicher, dass auch über einen längeren Zeitraum hinweg keine Verschmutzung der Folie durch Partikel oder Flüssigkeiten erfolgt, wodurch die Menge an in das Fluid eingestrahlter UV Strahlung reduziert würde. Die verringerte Benetzbarkeit mit Flüssigkeit bewirkt, dass sich keine die UV-Strahlung absorbierende oder streuenden Flüssigkeitstropfen auf der Folie absetzen. Auch hierdurch wird der gleichbleibende Eintrag von UV-Strahlung in das zu desinfizierende Fluid und damit die Desinfektionsleistung verbessert.

In der erfindungsgemäßen Desinfektionsvorrichtung ist vorgesehen, dass der Fluidleitungsabschnitt relativ zu der UV-Einheit gesehen von dieser beabstandet angeordnet ist. Alternativ hierzu besteht selbstverständlich auch die Möglichkeit, dass der Fluidleitungsabschnitt auf der UV-Einheit aufgelegt, aufgeklebt oder aufgeschrumpft ist, diese somit unmittelbar berührt. Die Entnehmbarkeit ist gegeben, da Fluidleitungsabschnitt und UV-Einheit auch in dieser Ausgestaltung in einfacher Art und Weise voneinander gelöst werden können.

Im Falle, dass nach längerer Verwendung eine Verringerung der Desinfektionsleistung festgestellt wird, wird es als günstig angesehen, wenn der Fluidleitungsabschnitt aus der Vorrichtung entnehmbar ausgebildet ist und gereinigt oder ausgetauscht werden kann. Hierdurch kann in einfacher Art und Weise das ursprüngliche Niveau der Desinfektionsleistung hergestellt werden, ohne einen Austausch der UV-Einheit durchführen zu müssen. Die Lebensdauer der Gesamtvorrichtung wird dadurch erhöht, die Wartung wesentlich vereinfacht.

Die erfindungsgemäße Desinfektionsvorrichtung zeichnet sich in einer bevorzugten Ausführungsform dadurch aus, dass sie einen Rohrkörper mit einer Innenwandung aufweist. In der bevorzugten Ausführungsform sind wenigstens eine erste UV-Einheit und der Fluidleitungsabschnitt innerhalb der Innenwandung angeordnet. Hierbei ist selbstverständlich auch eine Ausgestaltung des Rohrkörpers mit konzentrisch ineinander angeordneten Rohrabschnitten möglich. Den inneren Rohrabschnitt bildet dabei der Fluidleitungsabschnitt, während der äußere Rohrabschnitt im Zwischenbereich die UV-Einheit aufnimmt. Auch in dieser Ausgestaltung wird eine vollständige Trennung von den inneren Teil des Rohrabschnittes bildendem Fluidleitungsabschnittes und UV-Einheit erreicht.

Erfindungsgemäß ist vorgesehen, dass ein Fluideintritt an einem ersten Ende des Rohrkörpers und ein Fluidaustritt an einem zweiten Ende des Rohrkörpers erfolgt. Dabei weist der Rohrkörper bevorzugt eine definierte Länge auf. Die UV-Einheit und der Fluidleitungsabschnitt können sich dann über die gesamte Länge oder einen Teilbereich des Rohrkörpers erstrecken. Je nach gewünschter Desinfektionsleistung kann die Größe der UV-Einheit eingerichtet werden. Auch die Länge des durchflossenen Fluidleitungsabschnittes beeinflusst unmittelbar die Dauer und damit Intensität der Desinfektion.

Eine bevorzugte Weiterbildung der Erfindung sieht vor, dass der Rohrkörper zweiteilig ausgebildet ist, wobei die UV-Einheit einen äußeren Teil bildet oder in dessen Innenwandung integriert oder mit dieser verbunden vorgesehen ist und der Fluidleitungsabschnitt einen zweiten konzentrisch mit dem ersten Teil angeordneten Teil bildet. Auch in diesem Fall, in dem die Desinfektionsvorrichtung als Rohrkörper oder als Schlauch ausgebildet ist, ist ein Fluideintritt an einem ersten Ende des Rohrkörpers oder Schlauches und ein Fluidaustritt an einem zweiten Ende des Rohrkörpers oder Schlauches vorgesehen.

In der Desinfektionsvorrichtung gemäß der vorliegenden Erfindung ist in einer vorteilhaften Ausgestaltung vorgesehen, dass das zu desinfizierende Fluid passiv in die Desinfektionsvorrichtung einströmt. Die Desinfektionsvorrichtung ist dabei Teil einer übergeordneten Anlage, die das Fluid, beispielsweise Luft oder Wasser, kontinuierlich umwälzt. Durch die Umwälzung wird das Einströmen des Fluid in die Desinfektionsvorrichtung betrieben und das desinfizierte Fluid nach dem Durchlauf der Desinfektionsvorrichtung wieder in den umgewälzten Fluidstrom eingespeist. In dieser Ausgestaltung bedarf es keiner weiteren Mittel, um einen kontinuierlichen Durchtritt des Fluid durch die Desinfektionsvorrichtung sicherzustellen. Eine alternative Ausgestaltung der Desinfektionsvorrichtung hingegen sieht eine aktive Fluidzuführung vor. In diesem Fall ist insbesondere wenigstens eine Ansaug- oder Umwälzvorrichtung vorgesehen, die jeweils der Desinfektionsvorrichtung zugeordnet oder in diese integriert vorgesehen ist. Die Ansaug- oder Umwälzvorrichtung stellt einen kontinuierlichen Fluidfluss durch die Desinfektionsvorrichtung und damit eine umfassende Desinfektion des Fluids sicher.

In einer ebenfalls von der Erfindung umfassten Ausführungsform der Desinfektionsvorrichtung ist vorgesehen, dass es sich bei dem Fluid um ein Gas, insbesondere Luft, bevorzugt Atemluft handelt. Das vorgenannte, gasförmige Fluid wird der Desinfektionsvorrichtung dabei aktiv über wenigstens ein Gebläse zugeführt. Das Gebläse ist der Desinfektionsvorrichtung zugeordnet oder in diese integriert vorgesehen. Bei dem Gebläse kann er sich beispielsweise um einen oder mehrere in der Desinfektionsvorrichtung angeordneten Ventilator(en) handeln.

Die erfindungsgemäße Desinfektionsvorrichtung weist bevorzugt einen Rohrkörper mit einer runden, ovalen oder mehreckigen Außenkontur auf. Ausgehend vom vorgesehenen Einsatzzweck, kann somit eine Anpassung der Desinfektionsvorrichtung erfolgen. Die Außenkontur kann dabei auf die der die Desinfektionsvorrichtung aufnehmenden bzw. integrierenden, übergeordneten Einheiten abgestimmt werden.

Die Anpassbarkeit der Desinfektionsvorrichtung an verschiedene Einsatzzwecke wird dadurch verbessert, dass ein Rohrkörper vorgesehen wird, der starr oder flexibel, insbesondere schlauchartig, bevorzugt als Flüssigkeitsschlauch ausgebildet ist. Diese Ausgestaltung erlaubt eine einfache Integration der Desinfektionsvorrichtung beispielsweise in Lüftungsgeräte, Lüftungsanlagen, Beatmungsgeräte oder luft- oder flüssigkeitsführende medizinische Geräte.

Erfindungsgemäß ist vorgesehen, dass die Desinfektionsvorrichtung in einer Ausführungsform als einzelnes, in sich abgeschlossenes Gerät, beispielsweise auch als Standgerät, ausgebildet ist. Ein derartiges Standgerät nimmt die Umgebungsluft auf, desinfiziert diese und gibt desinfizierte Luft wieder an einen Raum ab. Ist die Desinfektionsvorrichtung zur Desinfektion von Flüssigkeiten vorgesehen, kann auch in diesem Fall eine Ausbildung als in sich abgeschlossenes Einzelgerät vorgesehen werden. Flüssigkeit wird in die Desinfektionsvorrichtung eingeleitet, dort desinfiziert und dann aus dem Gerät ausgeleitet. Die Zuführung kann in diesem Fall aktiv oder passiv erfolgen, wie zuvor bereits ausgeführt.

Eine alternative Ausführungsform sieht vor, dass die Desinfektionsvorrichtung in eine übergeordnete Raumlüftungsanlage integriert wird. In diesem Fall wird der durch die Raumlüftungsanlage erzeugte Luftstrom in die Desinfektionsvorrichtung eingeleitet, dort desinfiziert und nach Durchlauf der Vorrichtung aus dieser ausgeleitet und in die übergeordnete Raumlüftungsanlage eingespeist. Es kann je nach Ausgestaltung der Desinfektionsvorrichtung eine Anpassung an die Raumlüftungsanlage und die anfallenden Luftmengen durch entsprechende Skalierung der Vorrichtungsgröße erfolgen.

In einer weiteren Ausführungsform der Desinfektionsvorrichtung ist vorgesehen, dass diese in eine Beatmungseinheit oder einen Beatmungsschlauch integriert wird oder einen Teilabschnitt eines Beatmungsgerätes oder -schlauches bildet. In dieser Ausführungsform kann die dem beatmeten Patienten zugeführte Luft desinfiziert werden. Hierdurch wird die während invasiver Beatmung oftmals auftretende Zusatzerkrankung, beispielsweise eine Entzündung der Atemwege durch in der zugeführten Atemluft unbeabsichtigt mitgeführte Keime wesentlich reduziert und die Erfolgsaussichten der Heilbehandlung bzw. Beatmung verbessert werden.

Die Belastung der zu desinfizierenden Fluide mit nicht durch UV-Strahlung inaktivierbare, mitgeführte Partikel wird dadurch reduziert, dass in der Desinfektionsvorrichtung zusätzlich wenigstens eine Filtereinheit für einströmendes und/oder ausströmendes Fluid vorgesehen ist. Hierdurch wird auch die Verschmutzung des Inneren des Fluidleitungsabschnittes durch die Partikel weiter reduziert.

Eine weitere signifikante Reduzierung der Keimzahl kann in einer vorteilhaften Weiterbildung der Erfindung dadurch erreicht werden, dass in der Desinfektionsvorrichtung zusätzlich eine sogenannte Sinusgeneratoreinheit zur Behandlung des einströmenden und/oder ausströmenden Fluids vorgesehen ist. Diese Sinusgeneratoreinheit ist im Fluidleitungsabschnitt der UV-Einheit vorgelagert oder nachgelagert vorgesehen. Als günstig wird angesehen, wenn die Sinusgeneratoreinheit im Bereich des Fluideintritts an einem ersten Ende des Rohrkörpers oder eines Fluidaustritts an einem zweiten Ende des Rohrkörpers virgesehn wird und/oder an einem Umfang des Rohrkörper angeordnet ist und deisen umschließt. Hierbei wird bevorzugt wenigstens ein Ferritring am Rohrkörper oder einem Fluidleitungsabschnitt angeordnet, über den bei elektrischer Beaufschlagung ein elektrisches Feld ausbildet und ein Signal bestehend aus hochfrequenten Schwingungen, die allmählich abklingen und sich dann in unterschiedlichen Intervallen wiederholen, eine "exponentiell abfallende Sinuswelle" in das Fluid eingeleitet wird. Die Sinusgenerator-Technologie kann Wasser behandeln und Keime (insbesondere Bakterien) abtöten, ohne chemische Desinfektionsmittel zu verwenden. Dies geschieht durch die Verwendung einer elektrischen Ladung und unter Nutzung von Osmose, um in Keime vorhandenes Wasser zu zwingen, die natürliche Funktionen zu stören und die Keime zu zerstören. Das durch den Ferritring erzeugte elektrische Feld lädt Keime auf und legt eine Ladung (entweder positiv oder negativ) an allen Keimen an, die durch die Einheit fließen bzw. im Fluid mitgeführt werden. Hieraus resultiert eine Schicht aus hochreinem Wasser, das eine "Benetzungsschicht" oder "Hydratationsschicht" um die Keime bildet. Sobald sich diese Wasserschicht gebildet hat, beginnt die Osmose zu wirken und drückt Wasser in das Zelllumen des Keims, wodurch dieser platzt und damit inaktiviert wird. In unabhängigen Tests konnte gezeigt werden, dass beispielsweise 99,99% der Staphylococcus Aureus- und E. Coli-Bakterien durch eine Sinusgenerator-Einheit abgetötet wurden. Im Zusammenhang mit der erfindungsgemäßen Desinfektionsvorrichtung kann somit ein Synergieeffekt aus UV-Desinfektion und Desinfektion mittels einer Sinusgenerator-Einheit erzielt und die Effizienz der Vorrichtung weiter verbessert werden. Beispielhafte Einsatzfelder sind Schwimmbäder, Kühltürme, Landwirtschaftliche Anwendungen: Ansammlung von Bakterien im Trinkwasser an Orten wie Hühnerfarmen und Fischfarmen, ohne hierauf beschränkt zu sein. Zusätzlich zur Desinfektion über UV-Strahlung ist in einer als vorteilhaft angesehenen Weiterbildung der Erfindung vorgesehen, dass bei gasförmigen Fluiden, insbesondere Luft, eine Beaufschlagung des einströmenden oder ausströmenden gasförmigen Fluids mit einem flüssigen Desinfektionsmittel erfolgt. Hierdurch wird die Desinfektion weiter verbessert und die durch die UV-Strahlung bereits erheblich reduzierte Keimbelastung weiter verringert. In diesem Zusammenhang wird es als besonders günstig angesehen, wenn eine Verneblung des flüssigen Desinfektionsmittels erfolgt, um hier die Bildung eines Aerosols zu erreichen. Die Verneblung erfolgt dabei insbesondere über einen Ultraschallvernebler und/oder eine Einspritzdüse. Ultraschallvernebler und/oder Einspritzdüse sind bevorzugt im ersten und/oder zweiten Bereich der Desinfektionsvorrichtung angeordnet und beaufschlagen entweder die eintretende, noch zu desinfizierende Luft oder die austretende bereits desinfizierte Luft mit dem Desinfektionsmittel. Als günstig wird in diesem Zusammenhang angesehen, wenn das Desinfektionsmittel im Aerosol eine Tröpfchengröße von zwischen 0,1 µm und 10 µm aufweist, ohne die Erfindung hierauf zu beschränken. Bei dem Desinfektionsmittel kann es sich insbesondere um eine hypochlorische Säure handeln bzw. kann die hypochlorische Säure Bestandteil des Desinfektionsmittels sein. Das Desinfektionsmittel ist jedoch nicht auf die vorgenannte Säure beschränkt. Gleichzeitig oder alternativ sind sämtliche Desinfektionsmittel verwendbar, die von Menschen oder Säugetieren bedenkenlos über die Atemwege aufgenommen und verstoffwechselt werden können und eine entsprechende Zulassung besitzen.

In einer als vorteilhaft angesehenen Weiterbildung der erfindungsgemäßen Desinfektionsvorrichtung, wie zuvor beschrieben, ist vorgesehen, dass die Beaufschlagung mit dem Aerosol permanent, gesteuert oder bedarfsweise erfolgt. So kann beispielsweise bei einer erkannten höheren Keimbelastung dauerhaft oder bedarfsweise Aerosol in das zu desinfizierende Fluid eingespeist werden. Als günstig wird in diesem Zusammenhang angesehen, wenn eine Vorrichtung zur Steuerung der Menge an Desinfektionsmittel und/oder Aerosol und/oder der Beaufschlagungsdauer der einströmenden oder ausströmenden Luft mit dem Desinfektionsmittel bzw. dem Aerosol vorgesehen ist. Als günstig wird dabei angesehen, wenn die Steuerung bevorzugt in eine übergeordnete Steuerung der Desinfektionsvorrichtung integriert ist.

Eine weitere Ausgestaltungsform der erfindungsgemäßen Desinfektionsvorrichtung sieht vor, dass das Fluid eine Flüssigkeit, insbesondere Wasser ist und eine Beaufschlagung der einströmenden oder ausströmenden Flüssigkeit mit einem Desinfektionsmittel vorgesehen ist, wobei das Desinfektionsmittel über ein Ventil oder eine Einspritzdüse der Flüssigkeit bevorzugt dosiert zugeführt wird.

Die Desinfektionsleistung der erfindungsgemäßen Desinfektionsvorrichtung wird dadurch weiter erhöht, dass eine gerichtete Streuung der von der UV-Einheit ausgehenden Strahlung erfolgt. Dabei wird es als günstig angesehen, wenn die Streuung über eine hinter der UV-Einheit angeordnete Strukturierung einer Reflexionsoberfläche erfolgt. Dies kann beispielsweise über eine eine Streustruktur aufweisende Folie und/oder über zwischen der UV-Einheit und dem Fluidleitungsabschnitt eingebrachte, bevorzugt unstrukturiert oder im Wesentlichen würfel-, quader- oder kegelförmig ausgebildete Streukörper erfolgen.

Eine bedarfsgerechte Steuerung der erfindungsgemäßen Desinfektionsvorrichtung kann in vorteilhafter Weise dadurch erfolgen, dass wenigstens ein Sensor zur Messung der Geschwindigkeit und/oder Menge des einströmenden und/oder ausströmenden Fluids vorgesehen ist. Bei diesem Sensor handelt es sich um einen handelsüblichen Strömungssensor, der in der Lage ist, Luftmengen und Strömungsgeschwindigkeiten zu messen. Die gemessenen Werte können dann in eine Steuerung eingespeist werden, um erforderlichenfalls eine Anpassung des Fluidstroms und der einströmenden Fluidmenge vorzunehmen. Dies beispielsweise über Erhöhung der Leistung eines übergeordneten Umwälzungssystems oder einer der Desinfektionsvorrichtung zugeordneten Ansaug- oder Umwälzvorrichtung. Zusätzlich oder alternativ kann auch wenigstens ein Sensor zur Messung der Qualität des einströmenden und/oder ausströmenden Fluids vorgesehen werden. Die gemessenen Sensorwerte dienen dann als Anhaltspunkt für die Bewertung der Desinfektionsleistung. Hieraus kann dann der Zeitpunkt für eine Wartung oder einen Austausch des Fluidleitungsabschnittes abgeleitet werden. Gleiches gilt für den in einer bevorzugten Weiterbildung der Erfindung vorgesehenen wenigstens einen Sensor zur Messung der UV-Strahlung, insbesondere der Strahlungsintensität und/oder der Wellenlänge.

Eine als vorteilhaft angesehene Ausgestaltungsform der erfindungsgemäßen Desinfektionsvorrichtung sieht vor, dass diese eine Einheit zur Steuerung der Vorrichtung, insbesondere zur Steuerung der ein- und/oder ausströmenden Fluidmenge und/oder der Strömungsgeschwindigkeit und/oder der Verwendungsdauer der Vorrichtung aufweist. Hierüber kann beispielsweise eingestellt werden, ob die Vorrichtung kontinuierlich in Betrieb ist oder nur zu bestimmten Zeiten oder bei bestimmten Indikationswerten, wie beispielsweise einer erhöhten Keimbelastung im zu desinfizierenden Fluid. Auch kann so die Fluidmenge gesteuert werden, die tatsächlich durch die Desinfektionsvorrichtung geleitet wird.

Gleichermaßen von erfinderischer Bedeutung ist eine Raumluftanlage, die mit einer wie zuvor beschriebenen Desinfektionsvorrichtung ausgestattet ist. Über eine solche Raumluftanlage kann die Desinfektion der Raumluft in einem oder mehreren Räumen oder Raumkomplexen erreicht werden. Hier kann eine Desinfektionsvorrichtung wie zuvor beschrieben beispielsweise in eine Klimatisierung des Raumes oder der Räume integriert werden und beispielsweise einer Klimaanlage vor- oder nachgeschaltet vorgesehen werden.

Ebenfalls von der Erfindung umfasst ist ein Beatmungsgerät, wie dieses beispielsweise in der intensivmedizinischen Behandlung von Patienten verwendet wird. Hierbei ist auch eine Integration einer Desinfektionsvorrichtung wie zuvor beschrieben vorgesehen. Die Ausgestaltung der erfindungsgemäßen Desinfektionsvorrichtung erlaubt eine besonders einfache und auch kostengünstige Wartung der Desinfektionseinheit eines solchen Beatmungsgerätes und stellt aufgrund der Ausgestaltung dennoch sicher, dass eine hohe Desinfektionsleistungen erzielt wird. Neben der alleinigen Behandlung der durchströmenden Beatmungsluft mittels UV-Strahlung kann zusätzlich eine Beaufschlagung der Beatmungsluft mit einem als Aerosol vorgesehenen Desinfektionsmittel erfolgen, dass in den Beatmungsluftstrom eingespeist wird. Die Beaufschlagung mit einem flüssigen Desinfektionsmittel verhindert auch, dass sich innerhalb der des Beatmungsgerätes Ansammlungen von Keimen aufbauen. Es ergibt sich hier wird somit ein synergistischer Effekt, da zum einen die das Beatmungsgerät durchströmende Luft mittels der UV-Strahlung keimfrei gemacht und gleichzeitig über das in die Atemluft eingegebene Desinfektionsmittel Infektionen im beatmeten Patienten reduziert und zugleich das Beatmungsgerät als solches keimfrei gehalten werden können. Denkbar ist auch, dass das Beatmungsgerät zu Reinigungszwecken zunächst mit desinfizierter Luft und anschließend mit desinfizierter Flüssigkeit bzw. mit einem Desinfektionsmittel gespült wird.

Die Erfindung umfasst auch die Verwendung einer wie zuvor beschriebenen Desinfektionsvorrichtung zur Desinfektion von Raumluft und von Beatmungsluft. Darüber hinaus ist auch die Verwendung der Desinfektionsvorrichtung zur Desinfektion von Flüssigkeiten, insbesondere Spülflüssigkeiten für medizinische Anwendungen umfasst.

Ein weiterer Aspekt der Erfindung ist es, eine medizinische Behandlungseinheit zur Verfügung zu stellen. Dabei handelt es sich beispielsweise um einen Behandlungsstuhl wie dieser beispielsweise in Zahnarztpraxen zum Einsatz kommt oder eine Behandlungsliege, wie diese in Therapieeinrichtungen, Arztpraxen oder Operationssälen Verwendung finden. Dabei ist vorgesehen, dass in diesen Behandlungseinheiten integriert wenigstens eine wie zuvor beschriebene Desinfektionsvorrichtung vorgesehen ist. Es kann somit zum einen desinfizierte Luft, zum anderen desinfizierte Flüssigkeit zur Verfügung gestellt werden. Die erfindungsgemäße Desinfektionsvorrichtung erlaubt dabei eine gleichbleibende Desinfektionsleistung bei besonderer Wartungsfreundlichkeit. Auch kann hier ein Synergieeffekt erzielt werden, da für die Spülung der Behandlungsbereiche am Patienten desinfizierte Flüssigkeit oder desinfizierte Luft zur Verfügung gestellt wird. Gleichzeitig kann durch Verwendung der erfindungsgemäßen Desinfektionsvorrichtung in einfacher Art und Weise eine Desinfektion der Geräte, die während der Behandlung verwendet werden, erreicht werden. Die Desinfektionsvorrichtung gemäß der vorliegenden Erfindung erzielt hier ein besseres und gleichbleibendes Desinfektionsergebnis bei erhöhter Wartungsfreundlichkeit und längerer Betriebsdauer sowie verlängerten Wartungsintervallen. Die Desinfektionsvorrichtung gemäß der Erfindung kann beispielsweise in Schläuche in der Behandlungseinheit integriert werden. Gleichzeitig oder alternativ hierzu kann die Desinfektionsvorrichtung der Behandlungseinheit zugeordnet werden und somit auf einfache und wartungsfreundliche Art und Weise eine Desinfektion des gesamten Behandlungsgebietes erzielen. Auch ist die Keimbelastung der jeweiligen Räume, in denen sich die Behandlungseinheit befindet, dadurch reduziert, dass die Umgebungsluft über die zuvor beschriebene Desinfektionsvorrichtung keimfrei gehalten wird.

Weitere Ausführungsformen und Vorteile der Erfindung ergeben sich anhand der nachfolgenden Beschreibung einzelner, jedoch nicht beschränkender Ausführungsbeispiele, die in den Figuren gezeigt sind.

Es zeigt
- **Fig. 1**: eine erfindungsgemäße Desinfektionsvorrichtung als Einzelgerät,
- **Fig. 2**: die Integration des der Desinfektionsvorrichtung in einen Schlauch,
- **Fig. 3**: eine Ausführungsform der erfindungsgemäßen Desinfektionsvorrichtung als Einheit zur Integration in eine übergeordnete Einheit und
- **Fig. 4**: eine medizinische Behandlungseinheit, hier einen Zahnarztstuhl, in den mehrere der erfindungsgemäßen Desinfektionsvorrichtungen integriert sind.

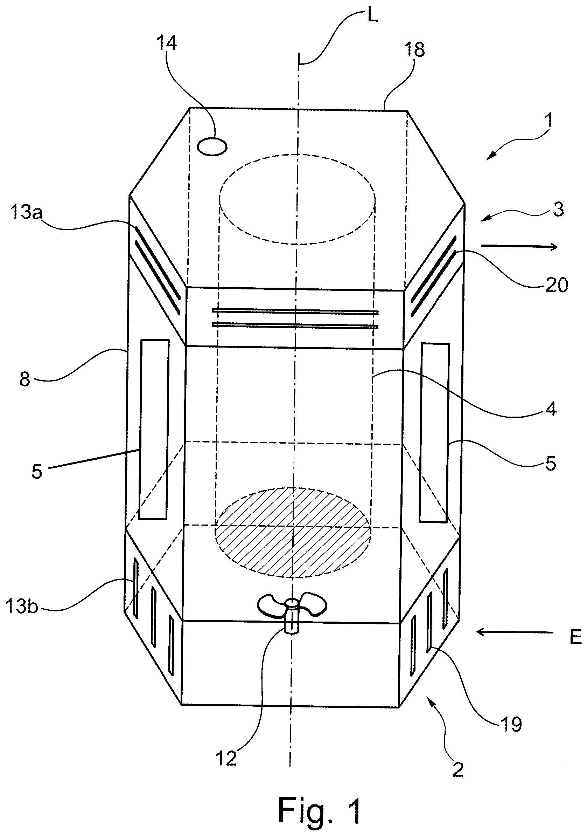

Fig. 1 zeigt eine Ausführungsform der erfindungsgemäßen Desinfektionsvorrichtung 1, die als eigenständiges Gerät ausgebildet ist. Die in Fig. 1 gezeigte Desinfektionsvorrichtung 1 dient zur Desinfektion von Raumluft und kann beispielsweise als Standgerät in einem Bereich eines Raumes aufgestellt werden, um im Umwälzverfahren die Raumluft zu desinfizieren. Die Desinfektionsvorrichtung 1 ist hierzu in einem Gehäuse 18 aufgenommen. Die zu desinfizierende Luft wird in einem ersten Bereich 2 des Gehäuses 18 in die Desinfektionsvorrichtung 1 in Einströmrichtung E eingesaugt. Zu diesem Zweck ist hier ein Lufteinlass 19 vorgesehen. Dem Lufteinlass 19 zugeordnet ist eine Filtereinheit 13b, die die Luft von Staubpartikeln und sonstigen Verunreinigungen befreit. Ebenfalls im ersten Bereich 2 des Gehäuses 18 vorgesehen ist ein Gebläse 12, dass das Einströmen der Luft in die Desinfektionsvorrichtung 1 bewirkt. Die Luft wird somit aktiv zugeführt. Nach dem Eintritt in das Gehäuse 18 durchströmt die Luft den zentral im Gehäuse 18 vorgesehenen, sich über die gesamte Längsachse L des Gehäuses 18 erstreckenden Fluidleitungsabschnitt 4, bis zu einem zweiten Bereich 3 der Desinfektionsvorrichtung 1, in dem ein Luftauslass 20 angeordnet ist. Auch diesem Luftauslass 20 ist eine Filtereinheit 13a zugeordnet, die eine weitere Filterung der desinfizierten Luft bewirkt. Der Reinheitsgrad der Luft wird somit weiter erhöht.

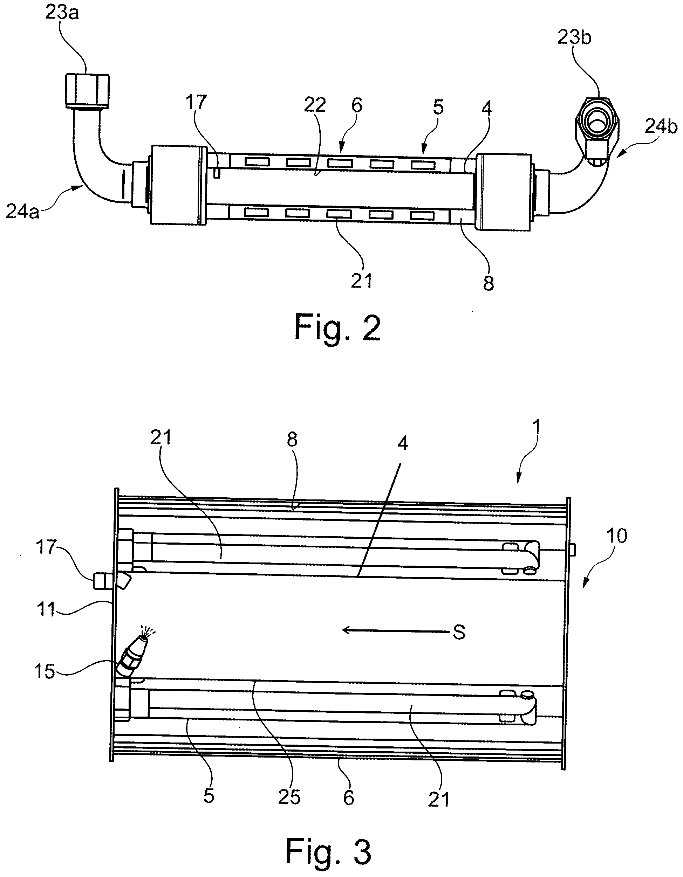

Konzentrisch zum Fluidleitungsabschnitt 4 und entlang der Innenwandung 8 des Gehäuses 18 ist in der Desinfektionsvorrichtung 1 eine UV-Einheit 5, bevorzugt eine UV-LED Einheit vorgesehen, die den Fluidleitungsabschnitt 4 auch vollständig umschließen kann. Die UV-Einheit 5 verfügt über mehrere Lichtelemente 21 (vgl. Fig. 2) die entlang der Innenwanderung 8 angeordnet sind. Die Lichtelemente 21 (vgl. Fig. 2) geben UV-Strahlung in einem Längenwellenbereich von beispielsweise 245 nm (UV-C) ab und bestrahlen die durch den Fluidleitungsabschnitt 4 strömende Luft, Dabei werden in der Luft mitgeführte Keime wie beispielsweise Viren, Pilze, Sporen oder Bakterien zu bis zu 99,9 Prozent abgetötet. Die so behandelte Luft tritt steril aus der Desinfektionsvorrichtung 1 bzw. dem Gehäuse 18 aus und wird in den Raum zurückgeführt.

Im Ausführungsbeispiel der Fig. 1 ist der Luftleitungsabschnitt 4 aus einem rohrförmigen Folienmaterial gebildet und beabstandet zu der UV-Einheit angeordnet. Durch den Luftleitungsabschnitt 4, der derart mit dem Lufteinlass 19 und dem Luftauslass 20 verbunden ist, dass hier keine Luft in das Gehäuse 18 einströmen kann wird verhindert, dass die UV-Einheit 5 mit der zu desinfizierenden Luft in Kontakt kommt. Der Luftleitungsabschnitt 4 schirmt die UV-Einheit 5 vollständig ab. Es wird auf diese Art und Weise verhindert, das in der eingeleiteten Luft mitgeführte Schmutzpartikel oder Flüssigkeitströpfchen zur UV-Einheit gelangen und sich dort niederschlagen. Der Luftleitungsabschnitt 4 bzw. die zu dessen Herstellung verwendete Folie weist eine Oberflächenbehandlung auf, die die Anhaftung von Partikeln bzw. Tröpfchen verhindert. Somit wird auch hier vermieden, dass es zu einer die Leistung der UV-Einheit 5 senkenden Verschmutzung der Folie kommt. Gleichwohl kann der Luftleitungsabschnitt 4 in einfacher Weise aus der Desinfektionsvorrichtung 1 entnommen und beispielsweise ausgetauscht oder gereinigt werden. Dies zum Beispiel im Zuge einer standardmäßigen Wartung der gesamten Vorrichtung. Durch die in Fig. 1 gezeigte Konfiguration der Desinfektionsvorrichtung wird die Wartungshäufigkeit der Anlage signifikant reduziert, da es zu keinen an Haftungen von Schmutz an den UV-Einheiten 5 kommen kann. Derartige Anhaftungen würden die Desinfektionsleistung signifikant verringern. Der Fluidleitungsabschnitt 4 ist transparent und durchlässig für UV-Strahlung ausgebildet. Das Gebläse 12 kann als Axial- oder Radialgebläse ausgebildet sein und ist sinnvollerweise innerhalb des Gehäuses 18 angeordnet. Somit wird die Luft durch die Filtereinheit 13b in den Desinfektionsvorrichtung 1 eingesaugt. Der Desinfektionsprozess kann kontinuierlich durchgeführt werden, auch während der Raum, in dem sich die Desinfektionsvorrichtung gemäß Fig. 1 befindet, genutzt wird.

In der in Fig. 1 gezeigten Ausführungsform weist die Vorrichtung zusätzlich einen schematisch dargestellten Vernebler 14 auf, über den ein flüssiges Desinfektionsmittel in Form eines Aerosols in die ausströmende Luft eingeführt wird. Hierdurch kann dauerhaft oder bedarfsweise eine zusätzliche Desinfektion der austretenden Luft erfolgen.

Fig. 2 zeigt eine weitere Ausführungsform der erfindungsgemäßen Desinfektionsvorrichtung 1. Diese ist mit einem Rohrkörper 6 ausgebildet, der in der Ausführungsform der Fig. 2 als Schlauch ausgebildet ist, somit eine flexible Ausgestaltung aufweist. Auch der Rohrkörper 6 verfügt über eine Innenwandung 8, auf der eine UV-Einheit 5 aufgelegt ist. Die UV-Einheit 5 verfügt über mehrere Lichtelemente 21, die UV-C Strahlung in das den Rohrkörper 6 durchströmende Fluid, beispielsweise ein Gas, Luft oder eine Flüssigkeit wie zum Beispiel Wasser, einträgt. Beim Durchströmen des Rohrkörper 6 erfolgt somit eine Abtötung von in dem Fluid mitgeführten Keimen. Die UV-Einheit 5 ist gegenüber dem durchströmenden Medium durch den konzentrisch zur UV-Einheit 5 in dem Rohrkörper 6 angeordneten Fluidleitungsabschnitt 4 vollständig abgeschirmt, Flüssigkeit kann somit die UV-Quelle erreichen. Somit wird verhindert, dass die UV-Einheit 5 durch das Fluid berührt wird und sich mitgeführte Partikel oder sonstige Verschmutzungen auf den Lichtelementen 21 absetzen. Verschmutzung der Lichtelemente 21 würde zu einer Reduzierung der eingestrahlten UV-Strahlung führen und damit die Desinfektionswirkung der gesamten Desinfektionsvorrichtung 1 reduzieren.

Der Fluidleitungsabschnitt 4 ist auch im Ausführungsbeispiel der Fig. 2 aus einem Folienschlauch gebildet. Anders als im Zusammenhang mit Fig. 1 beschrieben ist dieser Folienschlauch jedoch fest mit der UV-Einheit 5 verbunden. Aufgrund der Durchlässigkeit für UV-Strahlung kommt es hierbei jedoch zu keiner Reduzierung der Desinfektionsleistung der Lichtelemente 21. Der Fluidleitungsabschnitt 4 weist auf seiner dem Fluid zugewandten Innenseite 22 eine Beschichtung auf, die die Anhaftung von Partikeln wirksam verhindert. Zur Verbindung von Fluidleitungsabschnitt 4 und UV-Einheit 5 wird die Folie auf die UV-Einheit 5 aufgeschrumpft. Die UV-Einheit wird somit luft- und flüssigkeitsdicht gegenüber dem den Rohrkörper 6 durchströmenden Fluid abgedichtet. Die gesamte Einheit aus Fluidleitungsabschnitt 4 und UV-Einheit 5 wird im Rohrkörper 6 eingesetzt. Die UV-Einheit 5 sowie der Fluidleitungsabschnitt 4 sind hinreichend flexibel ausgebildet, so dass eine volle Flexibilität des als Schlauch ausgebildeten Rohrkörper 6 gewährleistet ist.

Zur Einbindung des Rohrkörper 6, der in Fig. 2 als Schlauch ausgebildet ist in ein übergeordnetes System (nicht dargestellt), verfügt dieser an beiden Enden 24a, b über Schraubverbindungen 23a, b. Selbstverständlich können diese auch als Steckverbinder oder sonstige geeignete Verbindungsmittel ausgebildet werden. Zur Messung der Durchflussrate weist die in Fig. 2 gezeigte Ausführungsform der Desinfektionsvorrichtung 1 zusätzlich einen Sensor 17 auf. Die ermittelnden Durchflusswerte werden an ein übergeordnetes Steuersystem weitergegeben, dass die Durchflussrate bzw. Durchflussmenge an zu desinfizierendem Fluid erforderlichenfalls anpasst, d.h. erhöht oder reduziert.

Fig. 3 zeigt eine weitere Ausführungsform der erfindungsgemäßen Desinfektionsvorrichtung 1. Diese ist ebenfalls in einem Rohrkörper 6 angeordnet. Im Ausführungsbeispiele der Fig. 3 ist dieser jedoch ein starres Rohr, in dessen Inneren die Desinfektionsvorrichtung 1 eingebracht ist. Dieses Rohr kann beispielsweise in ein übergeordnetes System wie eine Raumklimatisierung oder eine Raumlüftungsanlage eingesetzt werden und dort die durchströmende Luft desinfizieren. Die zu desinfizierende Luft tritt an einem ersten Ende 10 in den Rohrkörper 6 ein und durchströmt diesen in axialer Richtung S. Der Fluidleitungsabschnitt 4 ist dabei konzentrisch zu einer Innenwandung 8 des Rohrkörpers 6 zwischen dem ersten Ende 10 und einem zweiten Ende 11 angeordnet und leitet den Luftstrom durch die Desinfektionsvorrichtung 1. Gleichzeitig schirmt der Fluidleitungsabschnitt 4, der aus einem transparenten und für UV-Strahlung durchlässigen Folienmaterial gebildet ist, die UV-Einheit 5, die an der Innenwandung 8 des Rohrkörpers 6 angeordnet ist und den Fluidleitungsabschnitt 4 an dessen Umfang 25 umgibt, vollständig gegenüber dem durchgeleiteten Fluid ab. Die UV-Einheit 5 weist mehrere Lichtelemente 21 auf, die UV-Strahlung abgeben und das den Fluidleitungsabschnitt 4 durchströmende Fluid desinfizieren.

Im Bereich des zweiten Ende 11 des Rohrkörper 6 und innerhalb des Fluidleitungsabschnittes 4 befindet sich eine Einspritzdüse 15, über die ein flüssiges Desinfektionsmittel in Aerosolform in den desinfizierten, gasförmigen Fluidstrom eingebracht werden kann. Durch die Beaufschlagung mit einem flüssigen Desinfektionsmittel kann dieses beispielsweise in einen Raum eingebracht werden, gleichzeitig wird die Sterilität des Fluid weiter erhöht.

Auch die Desinfektionsvorrichtung 1 in Fig. 3 gezeigt verfügt über einen Sensor 17, der die Durchstromrate misst. Andere Sensorarten können ebenfalls vorgesehen werden, entweder alternativ oder zusätzlich. So können beispielsweise Sensoren vorgesehen werden, die die Qualität und Reinheit des durchströmenden Fluid nach der Desinfektion messen. Gleichzeitig besteht auch die Möglichkeit die Temperatur und den Feuchtigkeitsgehalt eines gasförmigen Fluids zu erfassen.

Die in Fig. 3 gezeigte Einheit kann beispielsweise in ein übergeordnetes Rohrleitungssystem einer Raumluftanlage eingebaut werden und auf einfache Art und Weise die Desinfektion der durchströmenden Luft sicherstellen. Der Fluidleitungsabschnitt 4 ist vollständig aus der Einheit entnehmbar und kann zu Wartungszwecken oder zur Reinigung ausgebaut werden. Der Fluidleitungsabschnitt 4 verhindert die Verschmutzung der UV-Einheit, deren Reinigung bzw. Austausch wesentlich arbeitsintensiver und kostspieliger wäre. Die in Fig. 3 gezeigte Einheit kann somit dazu beitragen, in Raumluftanlagen die Wartungsintervalle zu verlängern und Wartungskosten zu reduzieren. Gleichzeitig gestaltet sich der Austausch wesentlich einfacher, da lediglich der Luftleitungsabschnitt 4 aus der Einheit entnommen und ersetzt bzw. gereinigt werden muss.

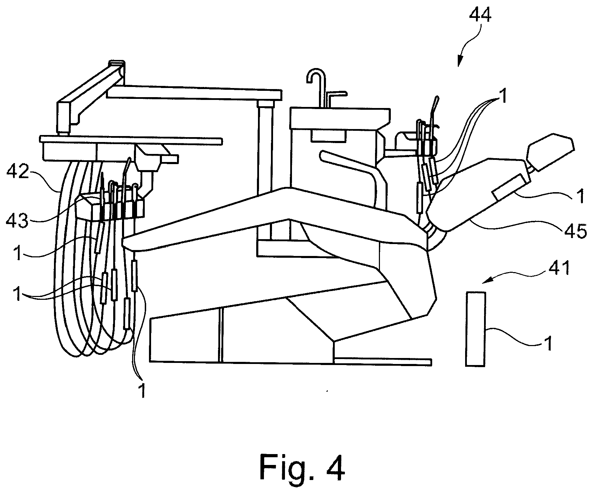

Fig. 4 zeigt eine medizinische Behandlungseinheit, hier einen Zahnarztstuhl 41, in den mehrere der zuvor beschriebenen Desinfektionsvorrichtungen 1 integriert sind. So sind in die Schlauchleitungen 42, die die zahnmedizinischen Geräte 43, wie beispielsweise Bohrer, mit für den Betrieb notwendiger Luft versorgen, Desinfektionsvorrichtungen 1 wie beispielsweise im Zusammenhang mit Fig. 2 beschrieben, integriert. Die für den Betrieb der zumeist druckluftbetriebenen Geräte notwendige Luft kann somit vor der Einleitung in die Geräte desinfiziert werden. Gleichzeitig besteht die Möglichkeit, die eingeführte Luft mit einem Desinfektionsmittel zu beaufschlagen, um damit eine Desinfektion des Inneren der zahnmedizinischen Geräte 43 zu erreichen. Hierdurch wird die Wartung und Reinigung der Geräte nach Verwendung wesentlich vereinfacht und die Ansammlung von Keimen, die durch die für den Betrieb der Geräte benötigte Luft mitgeführt werden, in deren Inneren wesentlich reduziert. Hieraus resultiert insgesamt eine Senkung der Keimbelastung und damit Übertragung auf den Patienten.

Im Behandlungsbereich des Zahnarztstuhls 41 sind ebenfalls mehrere Geräte 44 dargestellt. Hierbei handelt sich beispielsweise um Spülgeräte, die während der Zahnbehandlung unmittelbar in den Mund des Patienten eingeführt werden und dort Flüssigkeiten abgeben, die den Behandlungsbereich befeuchten oder spülen. Auch diesen Geräten 44 sind Desinfektionsvorrichtungen 1 wie zuvor beschrieben beigegeben. Mit diesen wird vornehmlich Flüssigkeit, das zum Spülen des Mundes des Patienten verwendet wird, sterilisiert. Die Desinfektionsvorrichtung 1 ist hier wie im Zusammenhang mit Fig. 2 beschrieben ausgestaltet und von einem flüssigen Medium, beispielsweise Wasser oder Spül- bzw. Desinfektionslösung durchflossen. Ebenfalls zur unmittelbaren Verwendung im Mund des Patienten bestimmt sind Sauger bzw. Gebläsevorrichtungen. Auch diesen ist eine wie zuvor beschriebene Desinfektionsvorrichtung 1 zugeordnet, bevorzugt in den das Behandlungsfluid, Luft oder Wasser, zuführenden Schlauch eingebracht. Das im Mund des Patienten verwendete Fluid wird zuverlässig desinfiziert und Übertragung von Keimen damit verhindert. Gleichzeitig besteht hier die Möglichkeit, wenn es sich um einen gasförmiges Fluid handelt, zusätzlich ein Desinfektionsmittel in Aerosolform in den Luftstrom einzuspeisen, um die Desinfektionswirkung weiter zu erhöhen.

Dem Zahnarztstuhl 41 unmittelbar zugeordnet ist ebenfalls eine weitere Desinfektionsvorrichtung 1 die in die Seitenlehne 45 des Zahnarztstuhles 41 integriert ist. Hieraus tritt bei der Behandlung und im Betrieb des Zahnarztstuhles 41 desinfizierte Luft aus und hält damit den gesamten Behandlungsbereich im Wesentlichen keimfrei.

Eine weitere Desinfektionsvorrichtung 1 kann in Form eines, wie im Zusammenhang mit Fig. 1 beschriebenen Standgerätes im Bereich des Zahnarztstuhles 41 vorgesehen werden und dort die Umgebungsluft im Behandlungsraum desinfizieren. Die Größe der Desinfektionsvorrichtung 1 bzw. des Standgerätes wird dabei auf die Raumgröße abgestimmt und bewirkt eine zuverlässige Desinfektion der gesamten Raumluft. Selbstverständlich besteht auch die Möglichkeit in ein übergeordnetes Raumlüftungssystem eine Desinfektionsvorrichtung wie beispielsweise im Zusammenhang mit Fig. 3 beschrieben, einzubauen und die gesamte Zahnarztpraxis mit desinfizierter Luft zu versorgen. Bedarfsweise besteht hier auch die Möglichkeit, dass der Raumluft ein zusätzliches Desinfektionsmittel zugegeben wird, dass in die desinfizierte Raumluft eingestreut und über die austretende, desinfizierte Luft in den Raum eingebracht wird.

Fig. 4 zeigt lediglich eine Ausgestaltungsform einer medizinischen Behandlungseinheit. Gleichzeitig besteht die Möglichkeit, entsprechende Ausgestaltungen auch bei Patientenliegen, beispielsweise in OP-Räumen vorzusehen. Auch hier ist ein hoher Bedarf an desinfizierten Fluiden, wie beispielsweise Gasen, Luft oder Flüssigkeit vorhanden. Gleichzeitig befinden sich in der Raumluft Keime, die auch mit UV-Strahlung abgetötet werden können. Wichtig ist es hierbei, dass stets eine gleichbleibende UV-Strahlungsleistung an die zu desinfizierenden Fluide abgegeben wird. Die erfindungsgemäße Desinfektionsvorrichtung 1 schafft auch hier Vorteile bei der Wartung und dem Betrieb der Desinfektionsvorrichtung 1, da die Verschmutzungsneigung reduziert und die Wartung wesentlich vereinfacht wird.

## Patentansprüche

1. Desinfektionsvorrichtung zur Desinfektion (1) von Fluiden, bevorzugt flüssigen oder gasförmigen Fluiden, wobei das zu desinfizierende Fluid in einem ersten Bereich (2) in die Vorrichtung (1) eintritt und in einem vom ersten Bereich (2) beabstandeten zweiten Bereich (3) aus der Vorrichtung (1) austritt und zwischen erstem und zweitem Bereich (2, 3) ein von dem Fluid durchströmbarer Fluidleitungsabschnitt (4) vorgesehen ist und die Vorrichtung (1) wenigstens eine dem Fluidleitungsabschnitt (4) zugeordnete UV-Einheit, bevorzugt UV-LED Einheit (5) zur Desinfektion des Fluids aufweist, der Fluidleitungsabschnitt (4) transparent und durchlässig für UV-Strahlung und aus der Vorrichtung entnehmbar ausgebildet ist und die UV-Einheit (5) gegenüber dem durchströmenden Fluid abschirmt, **dadurch gekennzeichnet, dass** der Fluidleitungsabschnitt (4) aus einem für UV-Strahlung durchlässigen Folienmaterial, insbesondere Kunststoffmaterial gebildet ist und eine Desinfektion des Fluids mittels UV-Strahlung ohne Berührung der UV-Einheit (5) beim Durchströmen des Fluidleitungsabschnittes (4) vorgesehen ist.

2. Desinfektionsvorrichtung (1) nach Anspruch 1, **dadurch gekennzeichnet, dass** das Folienmaterial eine Materialstärke von zwischen 60 µm und 300 µm, insbesondere von 150 µm aufweist und insbesondere eine Durchlässigkeit für die von der UV-Einheit (5) abgegebenen UV-Strahlung von zwischen 80% und 99% aufweist, und wobei das Folienmaterial insbesondere selbstreinigend ausgebildet ist, bevorzugt eine geringe Benetzbarkeit mit Flüssigkeit aufweist und/oder eine verringerte Anhaftung von Schmutzpartikeln zulässt.

3. Desinfektionsvorrichtung (1) nach einem der vorhergehenden Ansprüche, **dadurch gekennzeichnet, dass** der Fluidleitungsabschnitt (4) relativ zu der UV-Einheit (5) gesehen beabstandet oder auf der UV-Einheit (5) aufgelegt, aufgeklebt oder aufgeschrumpft angeordnet ist.

4. Desinfektionsvorrichtung (1) nach einem der vorhergehenden Ansprüche, **gekennzeichnet durch** einen Rohrkörper (6), mit einer Innenwandung (8), wobei wenigstens eine erste UV-Einheit (5) und der Fluidleitungsabschnitt (4) innerhalb der Innenwandung (8) angeordnet sind und bevorzugt ein Fluideintritt an einem ersten Ende des Rohrkörpers (6) und ein Fluidaustritt an einem zweiten Ende des Rohrkörpers (6) vorgesehen ist, wobei der Rohrkörper (6) insbesondere eine runde, ovale oder mehreckige Außenkontur aufweist und starr oder flexibel, insbesondere schlauchartig, bevorzugt als Flüssigkeitsschlauch ausgebildet ist.

5. Desinfektionsvorrichtung (1) nach Anspruch 4, **dadurch gekennzeichnet, dass** der Rohrkörper (6) eine definierte Länge aufweist und sich die UV-Einheit (5) und der Fluidleitungsabschnitt (4) über die gesamte Länge oder über einen Teilbereich des Rohrkörpers (6) erstrecken oder der Rohrköper (6) zweiteilig ausgebildet ist, wobei die UV-Einheit (5) einen äußeren Teil bildet oder in dessen Innenwandung (8) integriert oder mit dieser verbunden vorgesehen ist und der Fluidleitungsabschnitt (4) einen zweiten konzentrisch mit dem ersten Teil angeordneten Teil bildet.

6. Desinfektionsvorrichtung (1) nach einem der vorhergehenden Ansprüche, **dadurch gekennzeichnet, dass** die Desinfektionsvorrichtung (1) als Rohrkörper (6) ausgebildet ist und ein Fluideintritt an einem ersten Ende (10) des Rohrkörpers (6) und ein Fluidaustritt an einem zweiten Ende (11) des Rohrkörpers (6) vorgesehen ist, wobei das zu desinfizierende Fluid passiv in die Desinfektionsvorrichtung (1) einströmt oder eine aktive Fluidzuführung, insbesondere wenigstens eine Ansaugvorrichtung, die bevorzugt jeweils der Desinfektionsvorrichtung (1) zugeordnet oder in diese integriert ist, vorgesehen ist.

7. Desinfektionsvorrichtung (1) nach einem der vorhergehenden Ansprüche, **dadurch gekennzeichnet, dass** das Fluid Luft ist und der Desinfektionsvorrichtung aktiv über wenigstens ein Gebläse (12) zugeführt wird, wobei das Gebläse der Desinfektionsvorrichtung (1) zugeordnet oder in diese integriert vorgesehen ist.

8. Desinfektionsvorrichtung (1) nach einem der vorhergehenden Ansprüche, **gekennzeichnet durch** eine Ausbildung als Standgerät oder eine Integration in eine Raumlüftungsanlage oder in eine Beatmungseinheit oder einen Teilabschnitt eines Beatmungsschlauches vorgesehen ist.

9. Desinfektionsvorrichtung (1) nach einem der vorhergehenden Ansprüche, **dadurch gekennzeichnet, dass** zusätzliche eine Filtereinheit (13a, b) für einströmendes oder ausströmendes Fluid und/oder eine Sinusgeneratoreinheit zur Behandlung des einströmenden oder ausströmenden Fluids vorgesehen ist, wobei die Sinusgeneratoreinheit im Bereich des Fluideintritts an einem ersten Ende des Rohrkörpers (6) oder eines Fluidaustritts an einem zweiten Ende des Rohrkörpers (6) oder einem Umfang des Rohrkörper (6) angeordnet ist.

10. Desinfektionsvorrichtung (1) nach einem der vorhergehenden Ansprüche, **dadurch gekennzeichnet, dass** das Fluid Luft ist und eine Beaufschlagung der einströmenden oder ausströmenden Luft mit einem flüssigen Desinfektionsmittel vorgesehen ist, wobei insbesondere eine Verneblung des flüssigen Desinfektionsmittels zur Bildung eines Aerosols (16) vorgesehen ist, wobei die Verneblung insbesondere über einen Ultraschallvernebler (14) oder eine Einspritzdüse (15) erfolgt und Ultraschallvernebler (14) oder Einspritzdüse (15) bevorzugt im ersten und/oder zweiten Bereich (2, 3) der Vorrichtung (1) angeordnet ist/sind und wobei das Desinfektionsmittel im Aerosol (16) bevorzugt eine Tröpfchengröße von zwischen 0,1 µm und 10 µm aufweist und das Desinfektionsmittel insbesondere bevorzugt aus einer hypochlorischen Säure besteht oder diese beinhaltet und die Beaufschlagung permanent, gesteuert oder bedarfsweise erfolgt und eine Vorrichtung zur Steuerung einer Menge an Desinfektionsmittel oder Aerosol (16) und/oder der Beaufschlagungsdauer der einströmenden oder ausströmenden Luft vorgesehen ist, wobei die Steuerung bevorzugt in eine Steuerung der Desinfektionsvorrichtung (1) integriert ist.

11. Desinfektionsvorrichtung (1) nach einem der vorhergehenden Ansprüche, **dadurch gekennzeichnet, dass** eine Streuung der von der UV-Einheit (5) ausgehenden Strahlung vorgesehen ist und die Streuung bevorzugt über eine hinter der UV-Einheit (5) angeordnete Strukturierung, insbesondere eine eine Streustruktur aufweisende Folie und/oder über zwischen der UV-Einheit (5) und dem Luftleitungsabschnitt (4) eingebrachte, bevorzugt unstrukturiert oder im Wesentlichen würfel-, quader- oder kegelförmig ausgebildete Streukörper erfolgt.

12. Desinfektionsvorrichtung (1) nach einem der vorhergehenden Ansprüche, **dadurch gekennzeichnet, dass** wenigstens ein Sensor (17) zur Messung der Geschwindigkeit und/oder Menge des einströmenden oder ausströmenden Fluids und/oder wenigstens ein Sensor (17) zur Messung der Qualität des einströmenden oder ausströmenden Fluids und/oder wenigstens ein Sensor (17) zur Messung der UV-Strahlung, insbesondere der Strahlungsintensität und/oder der Wellenlänge vorgesehen ist.

13. Desinfektionsvorrichtung (1) nach einem der vorhergehenden Ansprüche, **dadurch gekennzeichnet, dass** eine Einheit zur Steuerung der Vorrichtung, insbesondere zur Steuerung der ein- und/oder ausströmenden Fluidmenge und/oder der Strömungsgeschwindigkeit und/oder der Verwendungsdauer der Vorrichtung vorgesehen ist.

14. Luftzuführungsanlage ausgestattet mit einer Desinfektionsvorrichtung (1) nach einem der Ansprüche 1 bis 13, wobei die Luftzuführungsanlage als Raumluftanlage oder Beatmungsgerät ausgebildet ist und eine Verwendung der Desinfektionsvorrichtung (1) zur Desinfektion von Raumluft oder Beatmungsluft.

15. Medizinische Behandlungseinheit, insbesondere Behandlungsstuhl oder Behandlungsliege aufweisend wenigstens eine Desinfektionsvorrichtung (1) nach einem der Ansprüche 1 bis 13.

## Claims

1. Disinfection device for the disinfection (1) of fluids, preferably liquid or gaseous fluids, wherein the fluid to be disinfected enters the device (1) in a first region (2) and exits the device (1) in a second region (3) spaced from the first region (2) and a fluid conduit section (4) through which the fluid can flow is provided between the first and second regions (2, 3) and the device (1) has at least one UV unit, preferably UV-LED unit (5), associated with the fluid conduit section (4), for disinfecting the fluid, the fluid conduit section (4) is transparent and permeable to UV radiation and removable from the device and shields the UV unit (5) from the fluid flowing through it, **characterised in that** the fluid conduit section (4) is formed from a film material, in particular plastic material, permeable to UV radiation and disinfection of the fluid by means of UV radiation is provided without contact with the UV unit (5) as it flows through the fluid conduit section (4).

2. Disinfection device (1) according to claim 1, **characterised in that** the film material has a material thickness of between 60 µm and 300 µm, in particular of 150 µm, and in particular has a permeability for the UV radiation emitted by the UV unit (5) of between 80% and 99%, and wherein the film material is in particular designed to be self-cleaning, preferably has a low wettability with liquid and/or permits reduced adhesion of dirt particles.

3. Disinfection device (1) according to one of the preceding claims, **characterised in that** the fluid conduit section (4) is spaced apart relative to the UV unit (5) or is placed, glued or shrunk onto the UV unit (5).

4. Disinfection device (1) according to one of the preceding claims, **characterised by** a tubular body (6) with an inner wall (8), wherein at least a first UV unit (5) and the fluid conduit section (4) are arranged within the inner wall (8) and preferably a fluid inlet is provided at a first end of the tubular body (6) and a fluid outlet is provided at a second end of the tubular body (6), wherein the tubular body (6) has in particular a round, oval or polygonal outer contour and is rigid or flexible, in particular hose-like, preferably in the form of a liquid hose.

5. Disinfection device (1) according to claim 4, **characterised in that** the tubular body (6) has a defined length and the UV unit (5) and the fluid conduit section (4) extend over the entire length or over a partial region of the tubular body (6) or the tubular body (6) is constructed in two parts, wherein the UV unit (5) forms an outer part or is integrated into the inner wall (8) thereof or is provided connected thereto and the fluid conduit section (4) forms a second part arranged concentrically with the first part.

6. Disinfection device (1) according to one of the preceding claims, **characterised in that** the disinfection device (1) is designed as a tubular body (6) and a fluid inlet is provided at a first end (10) of the tubular body (6) and a fluid outlet is provided at a second end (11) of the tubular body (6), wherein the fluid to be disinfected flows passively into the disinfection device (1) or an active fluid supply, in particular at least one suction device, which is preferably assigned to or integrated into the disinfection device (1), is provided.

7. Disinfection device (1) according to one of the preceding claims, **characterised in that** the fluid is air and is actively supplied to the disinfection device via at least one blower (12), the blower being associated with the disinfection device (1) or being provided integrated therein.

8. Disinfection device (1) according to one of the preceding claims, **characterised by** a design as a stand-alone device or an integration into a room ventilation system or into a ventilation unit or a section of a ventilation tube.

9. Disinfection device (1) according to one of the preceding claims, **characterised in that** a filter unit (13a, b) for inflowing or outflowing fluid and/or a sine wave generator unit for treating the inflowing or outflowing fluid is additionally provided, wherein the sine wave generator unit is arranged in the region of the fluid inlet at a first end of the tubular body (6) or of a fluid outlet at a second end of the tubular body (6) or a circumference of the tubular body (6).

10. Disinfection device (1) according to one of the preceding claims, **characterised in that** the fluid is air and the inflowing or outflowing air is provided with a liquid disinfectant, wherein in particular nebulisation of the liquid disinfectant is provided to form an aerosol (16), wherein the nebulisation takes place in particular via an ultrasonic nebuliser (14) or an injection nozzle (15) and the ultrasonic nebuliser (14) or injection nozzle (15) is preferably arranged in the first and/or second region (2, 3) of the device (1) and wherein the disinfectant in the aerosol (16) preferably has a droplet size of between 0.1 µm and 10 µm and the disinfectant in particular preferably consists of or contains a hypochlorous acid and the application is permanent, controlled or on-demand and a device (1) is is provided for controlling a quantity of disinfectant or aerosol (16) and/or the exposure time of the incoming or outgoing air, wherein the control is preferably integrated into a control of the disinfection device (1).

11. Disinfection device (1) according to one of the preceding claims, **characterised in that** a scattering of the radiation emitted by the UV unit (5) is provided and the scattering preferably takes place via a structuring arranged behind the UV unit (5), in particular a foil having a scattering structure and/or via scattering bodies introduced between the UV unit (5) and the air duct section (4) and preferably unstructured or essentially cuboidal, cuboidal or conical in shape.

12. Disinfection device (1) according to one of the preceding claims, **characterised in that** at least one sensor (17) is provided for measuring the speed and/or quantity of the inflowing or outflowing fluid and/or at least one sensor (17) is provided for measuring the quality of the inflowing or outflowing fluid and/or at least one sensor (17) is provided for measuring the UV radiation, in particular the radiation intensity and/or the wavelength.

13. Disinfection device (1) according to one of the preceding claims, **characterised in that** a unit is provided for controlling the device, in particular for controlling the amount of fluid flowing in and/or out and/or the flow rate and/or the duration of use of the device.

14. Air supply system equipped with a disinfection device (1) according to one of claims 1 to 13, wherein the air supply system is designed as a room air system or respiratory device and a use of the disinfection device (1) for disinfecting room air or respiratory air.

15. Medical treatment unit, in particular treatment chair or treatment bed, comprising at least one disinfection device (1) according to one of claims 1 to 13.

## Revendications

1. Dispositif de désinfection pour la désinfection (1) de fluides, de préférence des fluides liquides ou gazeux, dans lequel le fluide à désinfecter entre dans le dispositif (1) dans une première région (2) et sort du dispositif (1) dans une deuxième région (3) espacée de la première région (2) et une section de conduite de fluide (4) à travers laquelle le fluide peut s'écouler est prévue entre la première et la deuxième région (2, 3) et le dispositif (1) a au moins une unité UV, de préférence une unité UV-LED (5), associée à la section de conduite de fluide (4), pour désinfecter le fluide, la section de conduite de fluide (4) est transparente et perméable au rayonnement UV et amovible du dispositif et protège l'unité UV (5) du fluide qui la traverse, **caractérisée en ce que** la section de conduite de fluide (4) est formée d'un matériau de film, en particulier un matériau plastique, perméable au rayonnement UV et la désinfection du fluide au moyen du rayonnement UV est assurée sans contact avec l'unité UV (5) lorsqu'il traverse la section de conduite de fluide (4).

2. Dispositif de désinfection (1) selon la revendication 1, **caractérisé en ce que** le matériau du film a une épaisseur comprise entre 60 µm et 300 µm, en particulier de 150 µm, et en particulier a une perméabilité au rayonnement UV émis par l'unité UV (5) comprise entre 80% et 99%, et dans lequel le matériau du film est en particulier conçu pour être autonettoyant, a de préférence une faible mouillabilité avec le liquide et/ou permet une adhérence réduite des particules de saleté.

3. Dispositif de désinfection (1) selon l'une des revendications précédentes, **caractérisé par le fait que** la section (4) de conduit de fluide est espacée par rapport à l'unité UV (5) ou est placée, collée ou rétractée sur l'unité UV (5).

4. Dispositif de désinfection (1) selon l'une des revendications précédentes, **caractérisé par** un corps tubulaire (6) avec une paroi intérieure (8), dans lequel au moins une première unité UV (5) et la section de conduite de fluide (4) sont disposées à l'intérieur de la paroi intérieure (8) et, de préférence, une entrée de fluide est prévue à une première extrémité du corps tubulaire (6) et une sortie de fluide est prévue à une deuxième extrémité du corps tubulaire (6), le corps tubulaire (6) a en particulier un contour extérieur rond, ovale ou polygonal et est rigide ou flexible, en particulier en forme de tuyau, de préférence sous la forme d'un tuyau à liquide.

5. Dispositif de désinfection (1) selon la revendication 4, **caractérisé en ce que** le corps tubulaire (6) a une longueur définie et l'unité UV (5) et la section de conduite de fluide (4) s'étendent sur toute la longueur ou sur une région partielle du corps tubulaire (6) ou le corps tubulaire (6) est construit en deux parties, dans lesquelles l'unité UV (5) forme une partie extérieure ou est intégrée dans la paroi intérieure (8) de celui-ci ou y est connectée et la section de conduite de fluide (4) forme une deuxième partie disposée concentriquement avec la première partie.

6. Dispositif de désinfection (1) selon l'une des revendications précédentes, **caractérisé en ce que** le dispositif de désinfection (1) est conçu comme un corps tubulaire (6) et une entrée de fluide est prévue à une première extrémité (10) du corps tubulaire (6) et une sortie de fluide est prévue à une deuxième extrémité (11) du corps tubulaire (6), le fluide à désinfecter s'écoule passive-ment dans l'appareil de désinfection (1) ou une alimentation active en fluide, en particulier au moins un dispositif d'aspiration, qui est de préférence assigné ou intégré à l'appareil de désinfection (1), est prévu.

7. Appareil de désinfection (1) selon l'une des revendications précédentes, **caractérisé par le fait que** le fluide est de l'air et qu'il est activement fourni à l'appareil de désinfection par l'intermédiaire d'au moins un ventilateur (12), le ventilateur étant associé à l'appareil de désinfection (1) ou y étant intégré.

8. Appareil de désinfection (1) selon l'une des revendications précédentes, **caractérisé par** une conception en tant qu'appareil autonome ou une intégration dans un système de ventilation de pièce ou dans une unité de ventilation ou une section d'un tube de ventilation.

9. Dispositif de désinfection (1) selon l'une des revendications précédentes, **caractérisé par le fait qu'**une unité de filtrage (13a, b) pour le fluide entrant ou sortant et/ou une unité de génération d'ondes sinusoïdales pour traiter le fluide entrant ou sortant est également fournie, l'unité de génération d'ondes sinusoïdales étant disposée dans la zone de l'entrée de fluide à une première extrémité du corps tubulaire (6) ou d'une sortie de fluide à une deuxième extrémité du corps tubulaire (6) ou sur une circonférence du corps tubulaire (6).

10. Dispositif de désinfection (1) selon l'une des revendications précédentes, **caractérisé par le fait que** le fluide est de l'air et que l'air entrant ou sortant est pourvu d'un désinfectant liquide, dans lequel en particulier la nébulisation du désinfectant liquide est prévue pour former un aérosol (16), dans lequel la nébulisation a lieu en particulier via un nébuliseur ultrasonique (14) ou une buse d'injection (15) et le nébuliseur ultrasonique (14) ou la buse d'injection (15) est de préférence disposé dans la première et/ou la deuxième région (2, 3) de l'appareil (1) et dans lequel le désinfectant dans l'aérosol (16) a de préférence une taille de gouttelette comprise entre 0.1 µm et 10 µm et le désinfectant, en particulier, consiste de préférence en un acide hypochloreux ou en contient et l'application est permanente, contrôlée ou à la demande et un dispositif (1) est prévu pour contrôler une quantité de désinfectant ou d'aérosol (16) et/ou le temps d'exposition de l'air entrant ou sortant, le contrôle étant de préférence intégré dans un contrôle de l'appareil de désinfection (1).

11. Dispositif de désinfection (1) selon l'une des revendications précédentes, **caractérisé en ce qu'**une diffusion du rayonnement émis par l'unité UV (5) est prévue et que la diffusion a lieu de préférence par l'intermédiaire d'une structure disposée derrière l'unité UV (5), en particulier une feuille ayant une structure de diffusion et/ou par l'intermédiaire de corps de diffusion introduits entre l'unité UV (5) et la section de conduit d'air (4) et de préférence non structurés ou de forme essentiellement cuboïdale, cuboïdale ou conique.

12. Dispositif de désinfection (1) selon l'une des revendications précédentes, **caractérisé en ce qu'**au moins un capteur (17) est prévu pour mesurer la vitesse et/ou la quantité du fluide entrant ou sortant et/ou au moins un capteur (17) est prévu pour mesurer la qualité du fluide entrant ou sortant et/ou au moins un capteur (17) est prévu pour mesurer le rayonnement UV, en particulier l'intensité du rayonnement et/ou la longueur d'onde.

13. Dispositif de désinfection (1) selon l'une des revendications précédentes, **caractérisé en ce qu'**une unité est prévue pour contrôler le dispositif, notamment pour contrôler la quantité de fluide entrant et/ou sortant et/ou le débit et/ou la durée d'utilisation du dispositif.

14. Système d'alimentation en air équipé d'un appareil de désinfection (1) selon l'une des revendications 1 à 13, dans lequel le système d'alimentation en air est conçu comme un système d'air ambiant ou un appareil respiratoire et dans lequel l'appareil de désinfection (1) est utilisé pour désinfecter l'air ambiant ou l'air respiratoire.

15. Unité de traitement médical, en particulier fauteuil ou lit de traitement, comprenant au moins un appareil de désinfection (1) selon l'une des revendications 1 à 13.
